**Europäisches Patentamt**

**European Patent Office**

**Office européen des brevets**

(19)

(11) Numéro de publication : **0 356 272 B1**

(12) # FASCICULE DE BREVET EUROPEEN

(45) Date de publication du fascicule du brevet :
**18.03.92 Bulletin 92/12**

(51) Int. Cl.$^5$ : **A61K 33/42,** // (A61K33/42,
33:14, 33:04)

(21) Numéro de dépôt : **89402050.2**

(22) Date de dépôt : **19.07.89**

(54) **Médicaments destinés à regulariser les sécrétions hormonales et à traiter la stérilité chez la femme et les animaux femelles.**

(30) Priorité : **02.08.88 FR 8810432**

(43) Date de publication de la demande :
**28.02.90 Bulletin 90/09**

(45) Mention de la délivrance du brevet :
**18.03.92 Bulletin 92/12**

(84) Etats contractants désignés :
**AT BE CH DE FR GB IT LI LU NL SE**

(56) Documents cités :
**DICTIONNAIRE VIDAL, 1961, page 1014,
O.V.P., Paris, FR**

(73) Titulaire : **Letourneur, Bernard
Route de Oizé Le Champ du Bourray
Guécélard
F-72230 Arnage (FR)**

(72) Inventeur : **Letourneur, Bernard
Route de Oizé Le Champ du Bourray
Guécélard
F-72230 Arnage (FR)**

(74) Mandataire : **Casalonga, Alain et al
BUREAU D.A. CASALONGA - JOSSE
Morassistrasse 8
W-8000 München 5 (DE)**

## Description

L'invention a pour objet un médicament permettant de régulariser les sécrétions hormonales chez l'homme et la femme et traiter la stérilité chez la femme ainsi que chez les animaux femelles.

Ce médicament permet de lutter contre la stérilité féminine en régularisant les règles, les sécrétions hormonales oestrogènes (folliculine ou oestrone) et les sécrétions de progestérone ainsi que le taux de la prolactine.

Le médicament selon l'invention contribue également à régulariser la sécrétion de TSH (thyréostimuline) par l'hypophyse et de ce fait combat l'hypothyroïdie notamment le manque de $T_3$ (triiodothrionine), de $T_4$ (thyroxine) et de $FT_4$ ($T_4$ libre).

Ce médicament intervient également dans la synthèse et la sécrétion de FSH (hormone folliculo-stimulante) hypophysère et dans la synthèse de LH (hormone lutéinisante).

La régularisation du taux de FSH rend possible l'action de LH au niveau ovarien.

L'invention a pour objet une composition médicamenteuse destinée à régulariser les sécrétions hormonales et à traiter la stérilité de la femme.

L'invention a également pour objet une composition médicamenteuse destinée à faire baisser le taux de prolactine, en particulier pour l'action du chlorure de calcium.

L'invention a encore pour objet une composition médicamenteuse permettant de traiter l'hypotyroïdie.

L'invention a également pour objet un médicament permettant de régulariser les règles chez la femme et les animaux femelles et de favoriser l'ovulation.

D'autres objets de l'invention apparaîtront à la lecture de la description et des exemples.

La composition médicamenteuse selon l'invention comprend à titre d'éléments actifs : (a) du soufre, (b) du chlorure de calcium $CaCl_2$ et (c) de l'anhydride phosphorique $P_2O_5$.

Le soufre se présente de préférence sous forme de soufre sublimé ou fleur de soufre.

Le rapport en poids (a) soufre : (b) chlorure de calcium est compris entre 1 : 0,5 et 1 : 2 et avantageusement de 1 : 1,5 à 1 : 1,7.

Le rapport en poids (a + b) : (c) anhydride phosphorique est compris entre 4,5 et 3,6 et avantageusement entre 4,1 et 4,5.

Un rapport (a) : (b): (c) avantageux est 1 : 1,6 : 0,6.

Selon une réalisation préférée de l'invention, on administre la composition médicamenteuse par voie orale.

On peut également prévoir une administration par voie rectale.

Lorsqu'elle est administrée par voie orale, la composition médicamenteuse peut comporter un ou plusieurs excipients de préférence en poudre ou ne pas comporter d'excipient et être conditionnée dans des gélules.

Lorsqu'elle comporte des excipients, la composition médicamenteuse peut être conditionnée sous forme de pilules, de comprimés, de dragées et sous toute autre forme appropriée.

La composition médicamenteuse peut contenir tout excipient couramment utilisé pour la préparation des médicaments.

On peut citer notamment :

– des excipients tels que l'amidon, les sucres, le mannitol, le lactose ; les agents liants tels que le carboxycellulose, le carboxyméthylcellulose et autres dérivés cellulosiques, les alginates, la gélatine et la polyvinylpyrrolidone ;

– les agents humidifiants tels que la glycérine ;

– les lubrifiants tels que le talc ;

– les parfums et les agents améliorant le goût.

Des formes galéniques particulièrement avantageuses pour la voie orale sont les gélules, les comprimés, les dragées, mais l'on peut administrer la composition médicamenteuse sous toute forme galénique destinée à la voie orale.

Pour les suppositoires à administrer par voie rectale, on peut utiliser des excipients tels que le beurre de cacao, les huiles hydrogénées, les huiles hydrogénées et polyoxyéthylénées, les glycérides semi-synthétiques solides, les polyoxyéthylèneglycols ainsi que les autres excipients habituellement employés pour la confection des suppositoires.

La dose journalière recommandée est de 2,5 à 3,8 g de préférence 3,2 g de composition médicamenteuse renfermant :

de 0,8 à 1,2 g de soufre.

de 1,3 à 1,9 g de chlorure de calcium, et

de 0,5 à 0,7 g d'anhydride phosphorique $P_2O_5$.

Une dose journalière avantageuse comporte :

1,0 g de soufre

2

1,6 g de chlorure de calcium

0,6 g d'anhydride phosphorique.

La posologie recommandée consiste à administrer la dose ci-dessus en deux fois sous forme d'une gélule ou comprimé à midi et d'une gélule ou comprimé le soir à absorber au milieu des repas.

Lorsque le traitement est destiné à traiter la stérilité féminine ou commence l'administration au 24ème jour à partir des règles précédentes et on administre également pendant les règles.

On contrôle l'action du médicament en effectuant le dosage d'oestradiol, de la progestérone, de l'hormone LH et de l'hormone FSH.

Pendant le traitement, il est préférable de ne pas absorber des médicaments contenant des antibiotiques, des anti-inflammatoires et des antalgiques anti-pyrétiques comme le paracétamol et l'aspirine qui peuvent avoir une influence défavorable sur l'action du médicament selon l'invention.

Il est également important de s'abstenir d'utiliser des tampons hygiéniques pendant le traitement.

La composition médicamenteuse selon l'invention a été testée sur l'animal ainsi que chez des hommes et femmes qui étaient volontaires.

Les exemples ci-après illustrent l'invention.

EXEMPLE I

Les résultats de l'analyse du volontaire féminin n° 1 avant le traitement et après un traitement de huit semaines sont les suivants :

| | Avant traitement<br><br>Analyse effectuée le 24ème jour après les règles | Après un mois de traitement<br>Analyse effectuée le 24ème jour après les règles | Norme<br><br>adulte<br><br>phase lutéale |
| --- | --- | --- | --- |
| Oestradiol plasmatique | 24,8 ng/l | 104 ng/l | 70 à 250 ng/l |
| Progestérone plasmatique | 0,4 µg/l | 13,4 µg/l | 4,7 à 20 µg/l |
| LH plasmatique | 9,4 ui/l | 2,1 ui/l | 1 à 9 ui/l |
| FSH plasmatique | | 4 ui/l | 1,5 à 12 ui/l |

On voit que le traitement a permis d'augmenter les teneurs en oestradiol et progestérone, à faire baisser la teneur en LH plasmatique et à régulariser la teneur en FSH plasmatique.

EXEMPLE 2

Chez le volontaire féminin n° 2 les dosages effectués le 24ème jour après le début des règles et avant le traitement ont indiqué les résultats suivants :

Oestradiol : 0,1 ng/ml
LH plasmatique : 24,72 mUI/ml
FSH plasmatique : 4,80 mUI/ml
Prolactine : 4,41 ng/ml

On constate une trop faible dose d'oestradiol et une dose trop forte de LH plasmatique.

Après deux mois de traitement, la dose d'oestradiol a augmenté à 340 ng/ml

EXEMPLE 3

Les résultats effectués le 4ème jour du dosage à partir du début des règles et avant le traitement chez le volontaire féminin n° 3 sont les suivants :
Oestradiol : 0,5 ng/ml
Progestérone inférieure à 0,1 ng/l
LH plasmatique : 13,50 ui/l
FSH plasmatique : 4,0 ui/l

Après six semaines de traitement et au 24ème jour après les règles, on constate que la dose d'oestradiol a augmenté à 250 ng/ml et la dose de progestérone a augmenté à 16 ng/l;

Le traitement a permis de régulariser les dose hormonales et ces trois volontaires ont pu devenir enceintes.

**Revendications**

1. Composition médicamenteuse destinée à régulariser les sécrétions hormonales et à traiter la stérilité chez la femme et chez les animaux femelles, comprenant à titre d'éléments actifs (a) du soufre, (b) du chlorure de calcium et, (c) de l'anhydride phosphorique $P_2O_5$ soit seul, soit en présence d'un ou plusieurs excipient(s).

2. Composition médicamenteuse selon la revendication 1, caractérisée par le fait que le rapport en poids a : b est compris entre 1 : 0,5 et 1 : 2 et le rapport en poids (a + b) : c est compris entre 4,5 et 3,6.

3. Composition médicamenteuse selon la revendication 2, caractérisée par le fait que le rapport en poids a : b : c est de 1 : 1,6 : 0,6.

4. Composition médicamenteuse selon l'une quelconque des revendications 1 à 3, caractérisée par le fait qu'elle contient un excipient solide et se présente sous forme de comprimés, de pilules, ou sous toute autre forme solide.

5. Composition médicamenteuse selon l'une quelconque des revendications 1 à 4, caractérisée par le fait que l'excipient est choisi parmi l'amidon, les sucres, les agents liants, les agents humidifiants, les lubrifiants, les parfums et les agents améliorant le gout.

6. Composition médicamenteuse selon l'une quelconque des revendications 1 à 3, caractérisée par le fait qu'elle ne contient pas d'excipient et se présente sous forme de gélules.

7. Composition médicamenteuse selon les revendications 1 à 3, caractérisée par le fait qu'elle se présente sous forme de suppositoires.

**Patentansprüche**

1. Medikamentöse Zusammensetzung zur Regelung der hormonellen Absonderung und zur Behandlung der Sterilität bei der Frau und bei Tierweibchen, enthaltend als aktive Bestandteile (a) Schwefel, (b) Calciumchlorid und (c) Phosphoranhydrid $P_2O_5$ entweder allein oder in Gegenwart eines oder mehrerer Zusatzstoffe.

2. Medikamentöse Zusammensetzung nach Anspruch 1, dadurch gekennzeichnet, daß das Gewichtsverhältnis a : b zwischen 1 : 0,5 und 1 : 2 liegt und daß das Gewichtsverhältnis (a+b) : c zwischen 4,5 und 3,6 liegt.

3. Medikamentöse Zusammensetzung nach Anspruch 2, dadurch gekennzeichnet, daß das Gewichtsverhältnis a : b : c 1 : 1,6 : 0,6 ist.

4. Medikamentöse Zusammensetzung nach irgendeinem der Ansprüche 1 bis 3, dadurch gekennzeichnet, daß sie einen festen Zusatzstoff enthält und sich in Form von Tabletten, Pillen oder unter jeder anderen festen Form darstellt.

5. Medikamentöse Zusammensetzung nach irgendeinem der Ansprüche 1 bis 4, dadurch gekennzeichnet, daß der Zusatzstoff aus Stärke, Zucker, Bindemitteln , Befeuchtungsmitteln , den Schmiermitteln, Parfums und Mitteln zur Verbesserung des Geschmacks ausgewählt sind.

6. Medikamentöse Zusammensetzung nach irgendeinem der Ansprüche 1 bis 3, dadurch gekennzeichnet, daß sie keinen Zusatzstoff enthält und sich in Form von Gelatinekapseln darstellt.

7. Medikamentöse Zusammensetzung nach irgendeinem der Ansprüche 1 bis 3, dadurch gekennzeichnet, daß sie sich in Form von Suppositorien darstellt.

## Claims

1. Medicinal composition for regulating hormonal secretions and for treating sterility in women and in female animals, comprising as active elements (a) sulphur, (b) calcium chloride and (c) phosphoric anhydride, $P_2O_5$ either alone or in the presence of one or more excipient(s).

2. Medicinal composition according to Claim 1, characterised in that the a:b weight ratio is between 1:0.5 and 1:2 and the (a + b):c weight ratio is between 4.5 and 3.6.

3. Medicinal composition according to Claim 2, characterised in that the a:b:c weight ratio is 1:1.6:0.6.

4. Medicinal composition according to any one of Claims 1 to 3, characterised in that it contains a solid excipient and is provided in the form of tablets, pills or in any other solid form.

5. Medicinal composition according to any one of Claims 1 to 4, characterised in that the excipient is chosen from starch, sugars, binding agents, moisturising agents, lubricants, perfumes and taste improving agents.

6. Medicinal composition according to any one of Claims 1 to 3, characterised in that it does not contain an excipient and is provided in the form of hard gelatin capsules.

7. Medicinal composition according to Claims 1 to 3, characterised in that it is provided in the form of suppositories.